# EUROPÄISCHE PATENTSCHRIFT

(11) **EP 2 855 656 B1**
(45) Veröffentlichungstag und Bekanntmachung des Hinweises auf die Patenterteilung: **18.09.2019**
(21) Anmeldenummer: 13729250.4
(22) Anmeldetag: 23.05.2013
(51) Int. Cl.: C12M 1/00

(54) **ZELLKULTURBEHÄLTER FÜR DEN EINMALGEBRAUCH**
CELL CULTURE CONTAINER FOR ONE-TIME USE
CONTENANT DE CULTURE CELLULAIRE, À USAGE UNIQUE

(30) Priorität: 24.05.2012 DE 102012010155
(43) Veröffentlichungstag der Anmeldung: 08.04.2015
(73) Patentinhaber: Sartorius Stedim Biotech GmbH, 37079 Göttingen (DE)
(72) Erfinder: ERL, Wolfgang, Ludwig, 93095 Hagelstadt (DE); SEIDL, Joseph, 94424 Arnstorf (DE)
(74) Vertreter: Müller-Boré & Partner Patentanwälte PartG mbB
(86) Internationale Anmeldenummer: PCT/EP2013/001525
(87) Internationale Veröffentlichungsnummer: WO 2013/174515

(56) Entgegenhaltungen:
- EP-A1- 1 072 274
- EP-A1- 1 430 831
- EP-A2- 1 481 693
- EP-A2- 1 598 086
- WO-A2-2006/034157
- DE-T2- 69 530 386
- DE-U1-202007 005 399
- US-A1- 2007 071 798

## Beschreibung

Die vorliegende Erfindung betrifft u. a. ein Verfahren zur Herstellung eines Zellkulturbehälters für den Einmalgebrauch.

Derartige Zellkulturbehälter sind aus dem Stand der Technik seit langer Zeit bekannt und sind mindestens teilweise aus Polymermaterial gefertigt. Die bekannten Zellkulturbehälter können mehrlagig aufgebaut sein, wobei die Innenlage, d.h. diejenige Lage, die mit den Zellkulturen in Berührung kommt, aus Polymermaterial gefertigt ist. In der Regel besteht zumindest diese Lage bzw. Schicht aus Polyethylen (PE) oder Ethylvinylacetat (EVA).

Ein Zellkulturbehälter für den Einmalgebrauch ist ein Behälter, der für die biopharmazeutische oder sonstige Produktion von Peptiden, Proteinen, Antikörpern oder sonstigen bakteriellen, mikrobiologischen oder zellulären Produkten in großen Mengen verwendet werden kann. Dieser Prozess wird auch als Fermentation bezeichnet, und für diesen Fermentationsprozess werden sogenannte Bioreaktoren verwendet. Im Zusammenhang mit der beschriebenen Erfindung kann man bei einem Zellkulturbehälter für den Einmalgebrauch, der üblicherweise aus Polymermaterial in Form eines Beutels für die Produktion großer Mengen biologischer Materialien konstruiert ist auch von einem Wegwerf-Bioreaktor sprechen. Im folgenden Text bezeichnet der Begriff Zellkulturbehälter jede Form eines Zellkulturbehälters für den Einmalgebrauch oder entsorgbaren Zellkulturbehälters oder Beutels oder Bioreaktors für die Produktion von Biomaterialien.

Vor dem Befüllen der bekannten Zellkulturbehälter, die insbesondere in Form von Zellkulturbeuteln vorliegen, werden diese zur Sterilisierung mit gamma-Strahlung behandelt. Dies ist nötig, damit sämtliche Mikroorganismen, die ein optimales Zellwachstum stören oder beeinflussen könnten, abgetötet werden. Die Sterilisierung läuft i. d. R. so ab, dass eine Vielzahl von Zellkulturbehältern in einen Strahlenapparat eingebracht wird. Anschließend werden die Zellkulturbehälter mit gamma-Strahlung behandelt.

Die Druckschrift DE 695 30 386 T offenbart ein Verfahren zum Sterilisieren eines aus Polymermaterialien hergestellten Behälters mit Gamma-Strahlung und einem Sauerstoff-Absorber.

Es hat sich gezeigt, dass bei einer Vielzahl der oben beschriebenen Zellkulturbehälter für den Einmalgebrauch das Wachstum von Zellkulturen oder bakteriellen oder mikrobiellen Kulturen beeinträchtigt ist. Es kommt nämlich häufig zu einer sogenannten "extended lag phase", nämlich einem verzögerten Wachstum der zu kultivierenden Zellen oder Organismen. Eine derartige "extended lag phase" ist insbesondere bei Bioprozessen zur Arzneimittelproduktion äußerst unerwünscht.

In der Vergangenheit wurde fast die gesamte bio-pharamazeutische Produktion in fest eingebauten Bioreaktoren in Form von Edelstahltanks durchgeführt. Traditionell wurde dabei Serum, normalerweise fetales Rinderserum, als Nahrungsergänzung verwendet, um Zellwachstum zu fördern.

Die Anwesenheit von Serum im Produktionsprozess behindert jedoch das sog. "downstream processing", d.h. die Gewinnung und Aufreinigung der Endprodukte. Deshalb geht seit etwa zehn Jahren das Bestreben der Hersteller ganz klar zur Vermeidung von Serum in Zellkulturen wegen des einfacheren "downstream processings". Zusätzlich führt das Entfernen von Serum aus den Zellkulturen zu einer wesentlichen Erleichterung der Zulassung und Zertifizierung biopharmazeutischer Produkte. In etwa demselben Zeitraum hat die pharmazeutische und biotechnologische Produktion in fixierten Stahltankanlagen deutlich abgenommen, wodurch Flexibilität gewonnen und Aufbauzeiten sowie Produktionskosten reduziert werden konnten. In fest eingebauten Anlagen wurde das Problem der "extended lag phase" kaum beobachtet. In Anwesenheit von Serum zeigen Zellkulturbehälter für den Einmalgebrauch keine "extended lag phase". Die Kombination von Zellkulturbehältern für den Einmalgebrauch mit serum-freien Medien, welche bei pharmazeutischen und biotechnologischen Produktionsprozessen immer mehr Anwendung findet ist jedoch regelmäßig betroffen vom Auftreten einer "extended lag phase". Bislang konnte dieses Problem nicht gelöst werden.

Der vorliegenden Erfindung liegt somit die Aufgabe zugrunde, die Nachteile der Einmal-Zellkulturbehälter aus dem Stand der Technik zu überwinden. Der Erfindung liegt insbesondere die Aufgabe zugrunde, Zellkulturbehälter für den Einmalgebrauch zur Verfügung zu stellen welche in ausreichendem Maße steril sind und bei denen eine "extended lag phase" nicht auftritt oder zumindest stark reduziert wird.

Diese Aufgabe wird gelöst durch ein Verfahren zur Herstellung gemäß Anspruch 1 eines mindestens teilweise aus Polymermaterial gefertigten Zellkulturbehälters für den Einmalgebrauch, wobei der Zellkulturbehälter zur Sterilisierung mit gamma-Strahlung in einer Bestrahlungsdosis von 25 bis 50 kGy behandelt wird, dadurch gekennzeichnet, dass der Zellkulturbehälter vor der Behandlung mit gamma-Strahlen mit Inertgas behandelt wird. Hierdurch kann die Menge an anwesendem Sauerstoff so gewählt werden, dass bei der Behandlung des Zellkulturbehälters mit gamma-Strahlen Aldehyde und Ketone in einer Menge von maximal 3 ppm entstehen, wobei die Behandlung mit gamma-Strahlung unter Anwesenheit von 0,05 bis 15% Sauerstoff erfolgt.

Die oben genannte Aufgabe wird ferner durch einen Zellkulturbehälter, der durch das erfindungsgemäße Verfahren hergestellt wurde, gelöst.

Die Erfinder haben überraschenderweise herausgefunden, dass bei der Behandlung der Zellkulturbehälter Aldehyde und Ketone entstehen und diese Aldehyde und Ketone für die beschriebene "extended lag phase" verantwortlich sind. Diese Aldehyde und Ketone entstehen offensichtlich durch eine Reaktion von bei der gamma-Bestrahlung entstehenden freien Radikalen mit Luftsauerstoff. Wie weiter unten dargelegt werden wird und auch den später beschriebenen Versuchsergebnissen und Tabellen bzw. Kurven zu entnehmen ist, sind diese entstehenden Aldehyde und Ketone in entsprechender Konzentration eindeutig für die extended lag phase verantwortlich.

Durch die Behandlung der Zellkulturbehälter mit Inertgas vor einer gamma-Bestrahlung kann eine Bildung von Ketonen und Aldehyden unterbunden werden. Als Inertgas bezeichnet man Gase, die sehr reaktionsträge (inert) sind, sich also an nur wenigen chemischen Reaktionen beteiligen. Beim erfindungsgemäßen Verfahren wird bevorzugt Stickstoff als Inertgas verwendet. Es können jedoch auch Edelgase, insbesondere Argon, oder Gemische aus Edelgasen und Stickstoff als Inertgas verwendet werden.

Bei einer bevorzugten Verfahrensvariante wird der Zellkulturbehälter vor einer gamma-Bestrahlung mit Inertgas gefüllt und/oder in einen verschließbaren, mit Inertgas gefüllten Behälter eingebracht. Beim Befüllen des Zellkulturbehälters mit Inertgas wird i. d. R. so vorgegangen, dass der Zellkulturbehälter mehrmals mit Inertgas befüllt und wieder entleert wird, bis er schließlich ein letztes Mal befüllt und so entleert wird, dass noch eine geringe Menge Inertgas im Behälter verbleibt. Dadurch wird sichergestellt, dass kein Luftsauerstoff mehr im Inneren des Zellkulturbehälters anwesend ist. Entscheidend ist, dass vor einer Behandlung mit gamma-Strahlung der Zellkulturbehälter mit Inertgas behandelt wird. Es ist jedoch bevorzugt, dass die gamma-Bestrahlung zeitnah auf die Behandlung mit Inertgas folgt. Vorzugsweise soll die gamma-Bestrahlung zur Sterilisierung innerhalb von 24 Stunden, beispielsweise innerhalb von 18 oder 12 Stunden, nach der Inertgasbehandlung erfolgen. Dies gewährleistet optimale Ergebnisse, was ein Zellwachstum im erfindungsgemäßen Zellkulturbehälter betrifft.

Wie oben erwähnt, kann der erfindungsgemäße Zellkulturbehälter vor einem Bestrahlen mit gamma-Strahlung in einen zweiten Behälter, insbesondere einen Beutel, welcher Inertgas enthält, eingebracht werden. Sowohl der erfindungsgemäße Zellkulturbehälter als auch dieser zweite Beutel weisen vorzugsweise Gassperrschichten auf. Der erfindungsgemäße Zellkulturbehälter weist eine äußere Schicht aus Polyethylen mit einer Dicke von 0,1 bis 0,5 mm sowie eine innere, mit der zu kultivierenden Zellkultur in Berührung kommende weitere Schicht aus Polyethylen oder EVA, die ebenfalls 0,1 bis 0,5 mm dick ist, auf. Gemäß der vorliegenden Erfindung betrifft der Begriff "innere Schicht" eine Lage, welche mit der zu kultivierenden Zellkultur in Kontakt steht, und der Begriff "äußere Schicht" eine weitere Lage, welche von dieser Schicht verschieden ist.

Die äußere Lage ist beispielsweise eine Lage, welche mit der Umwelt in Kontakt steht, also die äußerste Lage des Zellkulturbehälters bildet.

Ein bevorzugter erfindungsgemäßer Zellkulturbehälter weist eine äußere Schicht aus Polyethylen mit einer Dicke von 0,15 bis 0,35 mm sowie eine innere, mit der zu kultivierenden Zellkultur in Berührung kommende weitere Schicht aus Polyethylen oder EVA auf, die ebenfalls 0,15 bis 0,35 mm dick ist. Gemäß einer besonders bevorzugen Ausführungsform der vorliegenden Erfindung weist der Zellkulturbehälter eine äußere Schicht aus Polyethylen mit einer Dicke von 0,18 bis 0,3 mm, beispielsweise 0,2 mm, sowie eine innere weitere Schicht aus Polyethylen oder EVA auf, die ebenfalls 0,18 bis 0,3 mm, beispielsweise 0,2 mm, dick ist.

Zwischen den beiden Polyethylenschichten befindet sich vorzugsweise eine Schicht aus Ethylvinylalkohol (EVOH), die als Gassperrschicht dient. Durch diese Gassperrschicht wird verhindert, dass Inertgas, das in den Zellkulturbehälter vor einer gamma-Bestrahlung eingebracht wurde, entweichen kann.

Wie bereits oben dargelegt, wird bei einem kompletten Ausschluss von Sauerstoff beim Bestrahlen mit gamma-Strahlung eine "extended lag phase" verhindert. Nun wurde ferner überraschend festgestellt, dass bei Anwesenheit von geringen Mengen an Sauerstoff und damit bei Anwesenheit geringer Mengen an Aldehyden und/oder Ketonen ein Zellwachstum sogar noch optimiert werden kann. Je nach Intensität der gamma-Strahlung, des verwendeten Polymermaterials des erfindungsgemäßen Zellkulturbehälters und der Art der zu kultivierenden Zellen kann die Bestrahlung mit gamma-Strahlung unter Anwesenheit von bis zu 15% Sauerstoff erfolgen. Wie bereits oben dargelegt, wird bei Anwesenheit von 0% Sauerstoff eine "extended lag phase" sicher verhindert und Zellen können ohne jegliche Beeinträchtigung wachsen.

Allerdings kann das Zellwachstum unter Anwesenheit bestimmter Mengen an Sauerstoff, die jedoch unterhalb des Sauerstoffgehalts der Luft liegen müssen, durch Entstehung geringer Mengen an Aldehyden und Ketonen verbessert werden. Um dies zu erreichen, wird die Menge von 0,05 bis 15% an anwesendem Sauerstoff so gewählt, dass beim Bestrahlen mit gamma-Strahlung in einer Bestrahlungsdosis von 25 bis 50 kGy Aldehyde und Ketone mit einer Konzentration von max. ca. 3 ppm entstehen. Bei Werten über 3 ppm kommt es i. d. R. bereits zur ungewünschten "extended lag phase".

Zur Steuerung eines solchen Effekts kann bei dem erfindungsgemäßen Verfahren der Zellkulturbehälter vor der Bestrahlung mit gamma-Strahlen mit Inertgas gespült und mit Inertgas und/oder einer definierten Menge an Sauerstoff, die unterhalb des Sauerstoffgehalts der Luft liegt, beschickt werden. Hierdurch kann die Entstehung von Aldehyden und Ketonen, und damit das Auftreten einer "extended lag phase" entweder verhindert werden, oder eine bestimmte Menge an Aldehyden und Ketonen kann entstehen, um dadurch das Zellwachstum in dem Zellkulturbehälter sogar zu verbessern.

Die vorliegende Erfindung betrifft somit in einer spezifischen Ausführungsform ein Verfahren zur Sterilisation eines mindestens teilweise aus Polymermaterial gefertigten Zellkulturbehälters für den Einmalgebrauch, wobei der Zellkulturbehälter vor der Bestrahlung mit gamma-Strahlung wie vorstehend beschrieben mit Inertgas behandelt wird, um die Entstehung von Aldehyden und Ketonen, die für das Auftreten einer "extended lag phase" verantwortlich sind, zu verhindern oder eine bestimmte Menge an Aldehyden und Ketonen entstehen zu lassen, um dadurch das Zellwachstum in dem Zellkulturbehälter zu verbessern.

Gemäß der vorliegenden Erfindung erfolgt die Bestrahlung mit gamma-Strahlung unter Anwesenheit von 0,05 bis 15% Sauerstoff. Wie bereits vorstehend dargelegt, beeinflusst neben der Sauerstoffkonzentration, bei der mit gamma-Strahlung bestrahlt wird, auch die Intensität der gamma-Strahlung selbst und das verwendete Polymermaterial des erfindungsgemäßen Zellkulturbehälters sowie die Art der zu kultivierenden Zellen die Menge der entstehenden Aldehyde und Ketone. Bei Bestrahlungsdosen von 25 bis 50 kGy beträgt der Sauerstoffgehalt während der Bestrahlung mit gamma-Strahlung 0,05 bis 15%. Als weitere Beispiele für einen bevorzugten Sauerstoffgehalt sind Bereiche von 0,05 bis 10%, 0,1 bis 9% oder 0,2 bis 8% zu nennen.

Bei den genannten entstehenden Aldehyden und Ketonen handelt es sich insbesondere um wasserlösliche Aldehyde und Ketone. Wasserunlösliche Aldehyde und Ketone haben auf das Zellwachsturn im erfindungsgemäßen Zellkulturbehälter geringere Auswirkungen, weil lipophile Komponenten mit höherer Kettenlänge nicht im gleichen Maße in Zellkulturmedien übertreten, da diese üblicherweise Wasser als Hauptbestandteil enthalten. Im Rahmen der vorliegenden Erfindung werden als wasserlösliche Aldehyde und Ketone solche verstanden, deren Wasserlöslichkeit bei 20°C mindestens 30 g/l beträgt.

Bei den Aldehyden sind insbesondere Formaldehyd, Acetaldehyd und Propionaldehyd von Bedeutung. Bei den Ketonen sind insbesondere Aceton, Butanon, Methylpropylketon sowie 3-Pentanon zu nennen.

Die vorliegende Erfindung betrifft auch einen Zellkulturbehälter für den Einmalgebrauch, herstellbar nach einem der Ansprüche 2 und 2.

Wie bereits oben dargelegt, enthält der erfindungsgemäße Zellkulturbehälter Aldehyde und Ketone, insbesondere wasserlösliche Aldehyde und Ketone, in einer Konzentration von 0,01 bis 3 ppm.

Insbesondere bei Anwendungen des erfindungsgemäßen Zellkulturbehälters im Arzneimittelbereich ist dieser vorzugsweise frei von Aldehyden und Ketonen, insbesondere von wasserlöslichen Aldehyden und Ketonen. Erfindungsgemäß wird unter "frei von Aldehyden und Ketonen" verstanden, dass der erfindungsgemäße Zellkulturbehälter Aldehyde und Ketone in einer Menge von 0 bis zu 0,01 ppm enthält. Im Arzneimittelsektor dürfen Aldehyde und Ketone oftmals auch wegen Vorschriften aus dem Arzneimittelgesetz nicht vorhanden sein. In diesem Falle wird der erfindungsgemäße Zellkulturbehälter unter Ausschluss von Sauerstoff mit gamma-Strahlung sterilisiert.

Die erfindungsgemäßen Zellkulturbehälter enthalten diese Aldehyde und Ketone mit einer Konzentration von ca. 0,01 bis ca. 3 ppm, insbesondere von 0,1 bis 1 ppm. In diesem Bereich ist überraschender Weise ein besonders gutes Zellwachstum zu beobachten.

Wie bereits oben erwähnt, ist der erfindungsgemäße Zellkulturbehälter vorzugsweise als mehrlagiger Beutel ausgebildet, der bis zu sieben Lagen, beispielsweise drei Lagen, umfassen kann. Die verschiedenen Lagen des erfindungsgemäßen Zellkulturbehälters können aus unterschiedlichen Materialien oder aus denselben Materialien gefertigt sein, wobei die äußere und die innere Lage vorzugsweise aus PE und/oder EVA bestehen. In dieser Konfiguration ist es weiterhin bevorzugt, dass eine von der äußeren und inneren Lage verschiedene Lage, die im Folgenden als eine "mittlere Lage" bezeichnet wird, eine Gassperrschicht, insbesondere eine EVOH-Lage ist. Gemäß einer bevorzugten Ausführungsform ist der erfindungsgemäße Zellkulturbehälter als dreilagiger Beutel ausgebildet, wobei die innere und äußere Schicht vorzugsweise aus PE oder EVA besteht und die mittlere Lage eine Gassperrschicht, insbesondere eine EVOH-Lage ist.

Vorzugsweise sind mindestens in einer inneren Schicht bzw. Lage des erfindungsgemäßen Zellkulturbehälters UV-Stabilisatoren oder sogenannte "Anti-rads" enthalten. "Anti-rads" sind Substanzen, die ähnlich wie UV-Stabilisatoren wirken und negative Effekte der gamma-Strahlung auf Polymermaterial verhindern oder reduzieren. Diese Stabilisatoren unterdrücken ebenfalls die Bildung von Aldehyden und Ketonen, indem sie eine Radikalbildung beim Bestrahlen des erfindungsgemäßen Zellkulturbehälters mit gamma-Strahlen unterdrücken. Beispiele für UV-Stabilisatoren sind 2,6-Di-tert-butylphenol sowie Butyl-hydroxytoluol (BHT). Diese Beispiele sollen den Bereich der gegenwärtigen Erfindung in keiner Weise beschränken. Zusätzlich kann eine Kombination von zwei oder mehr UV-Stabilisatoren und/oder "Anti-rads" für die Produktion derartiger Zellkulturbehälter verwendet werden.

Die in der Polymerchemie üblicherweise verwendeten UV-Stabilisatoren und Anti-rads mit Schwefel- oder Stickstoffmolekülen in der Ringstruktur verbieten sich für eine Anwendung von Polymeren in der Zellkultur trotz ihrer überlegenen Wirkungsweise, weil aus diesen Molekülen unter Einwirkung von gamma-Strahlen Sulfit- und Nitrosoverbindungen entstehen können, welche eine hohe zytotoxische Wirkung besitzen.

Die hierin angegebenen Konzentrationen und Konzentrationsbereiche in "ppm" ergeben für unterschiedliche Aldehyde und Ketone zum Teil gegenläufige Konzentrationen in Mol/Liter, was sich durch das unterschiedliche Molekulargewicht und die unterschiedlichen Dichten von z.B. Aceton und Formaldehyd erklären lässt. Die zu beobachtende gegensätzliche Tendenz bei ppm und µM führt deshalb zur Angabe von Konzentrationsbereichen, die in keiner Weise als ausschließlich oder absolut betrachtet werden sollen. Vielmehr kann durch diese Angaben eine Konzentration gemeint sein, die sich im Bereich von +/- 10% bis 20% der angegebenen absoluten Grenzwerte bewegt.

Wie vorstehend beschrieben, basiert die vorliegende Erfindung zum einen auf der Erkenntnis, dass Aldehyde und Ketone, die während der Bestrahlung mit gamma-Strahlen im Rahmen eines Sterilisierungsverfahrens eines Zellkulturbehälters entstehen, für die beschriebene "extended lag phase" verantwortlich sind. Darüber hinaus basiert die vorliegende Erfindung auf der Erkenntnis, dass bei Anwesenheit von geringen Mengen an Sauerstoff und damit bei Anwesenheit geringer Mengen an Aldehyden und/oder Ketonen ein Zellwachstum sogar noch optimiert werden kann. Dadurch ist es möglich, eine effektivere Zellkultivierung zu ermöglichen. Die vorliegende Erfindung stellt daher auch ein Verfahren zur Zellkultivierung bereit, bei dem einer Zellsuspension wasserlösliche Aldehyde und/oder Ketone, ausgewählt aus der Gruppe, bestehend aus Formaldehyd, Acetaldehyd, Propionaldehyd, Aceton, Butanon, 2-Pentanon und 3-Pentanon, in einer Konzentration von ca. 0,01 bis 3 ppm, bevorzugt von 0,1 bis 1 ppm zugesetzt werden.

### Beispiele:

### 1. Nachweis des Einflusses von Aldehyden und Ketonen auf das Zellwachstum:

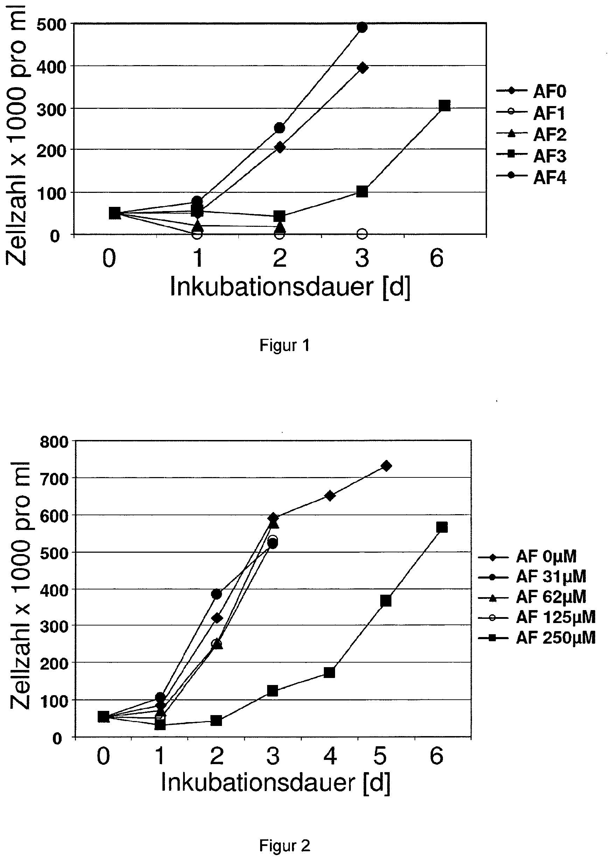

Fünf Proben einer Zellsuspension aus CHO AA8-Luc-Zellen (Zellen aus Ovarien chinesischer Hamster) und einem Medium (FMX-8), dessen Zusammensetzung in Tabelle 1 angegeben ist, wurden mit unterschiedlichen Mengen (weiter Konzentrationsbereich) an Aceton und Formaldehyd inkubiert. In der **Figur 1** ist das unterschiedliche Zellwachstum dargestellt:
AFO ist eine Kontrolle ohne Zugabe von Aceton oder Formaldehyd (= FMX-8 Medium). Hier ist ein normales, ungestörtes Wachstum zu beobachten.
AF1 entspricht 250 ppm Aceton und 250 ppm Formaldehyd (entspricht 4,3 mM Aceton und 8,325 mM Formaldehyd). Bei dieser Konzentration sterben alle Zellen ab.
AF2 entspricht 25 ppm Aceton und 25 ppm Formaldehyd (entspricht 430 µM Aceton und 832,5 µM Formaldehyd). Auch hier sterben alle Zellen ab.
AF3 entspricht 2,5 ppm Aceton und 2,5 ppm Formaldehyd (entspricht 43 µM Aceton und 83,25 µM Formaldehyd). Hier ist klar eine "extended lag phase" zu beobachten.
AF4 entspricht 0,25 ppm Aceton und 0,25 ppm Formaldehyd (entspricht 4,3 µM Aceton und 8,325 µM Formaldehyd). Hier ist ein verbessertes Wachstum zu beobachten.

Die beiden entscheidenden Kurven sind AF3 und AF4. Diese Ansätze zeigen eindeutig die unterschiedliche Wirkung von Aldehyd und Keton auf das Zellwachstum. Ist die Menge an zugesetztem Aceton/Formaldehyd gering, so zeigt sich gegenüber der Kontrolle AFO (Abwesenheit von Aldehyd und Keton) sogar ein verbessertes Wachstum. Bei AF3 ist eindeutig die sogenannte "extended lag phase" zu sehen, die häufig bei den Zellkulturbehältern aus dem Stand der Technik vorkommt. Beim Bestrahlen mit gamma-Strahlung entsteht in der Regel eine Menge an Aldehyden und Ketonen, die im Bereich von AF3 liegen. Innerhalb der ersten drei bis vier Tage Inkubationszeit der Zellkulturen ist hier nur ein äußerst geringes Zellwachstum zu erkennen. Erst nach dem dritten Tag kommt es zu einer Steigerung des Zellwachstums. Diese Erscheinung wird als "extended lag phase" bezeichnet.

**Figur 2** zeigt die Ergebnisse aus einem weiteren Durchgang mit fünf verschiedenen Zellkulturansätzen mit unterschiedlichen Konzentrationen (enger Konzentrationsbereich) an Aceton und Formaldehyd.

Zur Überprüfung der Proliferationssteigernden Wirkung von geringen Mengen an Aldehyden und Ketonen wurde eine weitere Versuchsreihe durchgeführt, die in **Figur 2** zusammengefasst ist. Hier wurde u. a. eine Menge von 31,25 µM an Formaldehyd und Aceton zu einer Zellsuspension aus CHO-Luc Zellen gegeben. Dies entspricht einer Konzentration von ca. 1 ppm an Aldehyden und Ketonen. Gegenüber der Kontrolle, bei der keine Aldehyde oder Ketone zugegeben wurden, zeigt diese Zellprobe eine gesteigerte Proliferation, was zusätzlich zu den Ergebnissen aus dem ersten Versuch über einen weiten Konzentrationsbereich den Wachstumsfördernden Effekt von Aldehyden und Ketonen in einer Konzentration von etwa 1 ppm für Suspensions-Zellkulturen verdeutlicht.

0 ist Kontrolle (= FMX-8 Medium)>> normales Wachstum
31,25 µM 5 Aceton (= 1,82 ppm) und 31,25 µM Formaldehyd (= 0,94 ppm)
>> verbessertes Wachstum
62,5 µM Aceton (= 3,64 ppm) und 62,5 µM Formaldehyd (= 1,88 ppm)
>> kein Effekt
125 µM Aceton (= 7,28 ppm) und 125 µM Formaldehyd (= 3,76 ppm)
>> kein Effekt
250 µM Aceton (= 14,56 ppm) und 250 µM Formaldehyd (= 7,52 ppm)
>> extended lag phase

Durch das unterschiedliche Molekulargewicht und die unterschiedliche Dichte von Aceton und Formaldehyd ist eine gegensätzliche Tendenz bei ppm und µM zu beobachten.

### 2. Bestimmung der Menge an Aldehyden und Ketonen, die bei der Bestrahlung von Zellkulturbehältern mit gamma-Strahlen entstehen:

Ein zu testender Kulturbeutel wurde mit 0,5 ml destilliertem Wasser pro cm² Beutelinnenfläche gefüllt. Das Wasser wurde 24 Stunden im Beutel belassen. Anschließend wurde die Menge an entstandenen wasserlöslichen Aldehyden und Ketonen gemessen. Mit dieser Methode können sämtliche wasserlösliche Aldehyde und Ketone erfasst werden. Die Messung der Aldehyde und Ketone kann beispielsweise mittels GC-MS durchgeführt werden.

### 3. Vergleich des Zellwachstums in unterschiedlich behandelten Zellkulturbeuteln:

PE-Folie (0,2 mm Dicke) wurde zu Beuteln verschweißt (Oberfläche der Innenseite ca. 100 cm². Die Beutel wurden jeweils 3x mit Luft, Sauerstoff (O₂), bzw. Stickstoff (N₂) gefüllt und entleert, so dass ein kleiner Rest des Gases im Beutel verblieb. Anschließend wurden die Beutel in 50 ml Zentrifugenröhrchen aus PP gegeben, diese wieder je 3x mit Luft, O₂ oder N₂ begast und dicht verschlossen. Dann wurden die Beutel mit 50 kGy gamma-Strahlung behandelt. Nach der Bestrahlung wurden die Beutel mit je 5 ml FMX-8 Medium steril befüllt und für 24 h bei 37°C inkubiert.

Das Medium wurde aus den jeweiligen Beuteln abgesaugt und je 1,5ml davon in einer 1:10 Verdünnung zu je 1,5 ml Zellsuspension gegeben, so dass eine Zellzahl von 50.000 pro ml bei Beginn der Inkubationen vorlag. Die Zellen wurden für 4 d bei 37°C und 5% CO₂ inkubiert und anschließend ausgezählt.

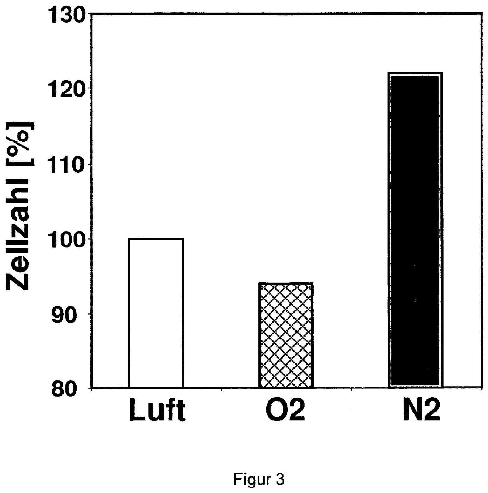

In **Figur 3** dargestellt ist das Zellwachstum, wobei das Medium aus mit Luft behandelten Beuteln als Kontrolle verwendet wurde und diese als 100% definiert wurde.

Eine Behandlung der Beutel mit O₂ führt zu einer Verzögerung des Zellwachstums. Eine Behandlung mit N₂ führt zu einer Verbesserung des Zellwachstums. Hier entstehen im Vergleich zu mit Luft oder reinem Sauerstoffbehandelten Beuteln kaum Aldehyde und Ketone (siehe Fig. 3).

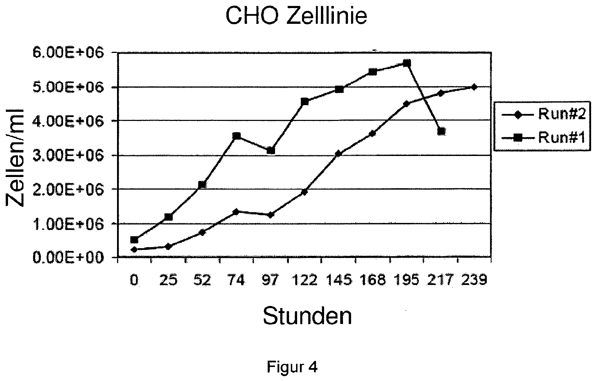

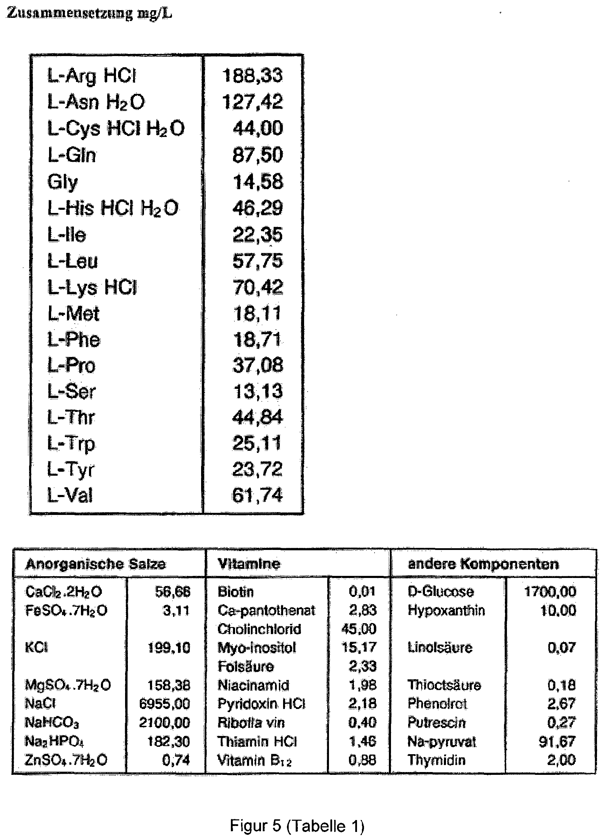

**Figur 4** zeigt das Zellwachstum in zwei verschiedenen Zellkulturbehältern aus Poly-ethylen, die beide mit gamma-Strahlung behandelt wurden, bevor sie mit Zellsuspensionen gefüllt wurden. Die beiden hier dargestellten Zellkulturbehälter stammen aus der gleichen Charge an Behältern, die gleichzeitig in einer Bestrahlungsvorrichtung mit gamma-Strahlen beschickt wurden. Der mit run #2 bezeichnete Behälter zeigt eine deutlich ausgeprägte "extended lag phase". Bei dem mit run #1 dargestellten Zellkulturbehälter ist dieser Effekt weniger ausgeprägt. Dies liegt im folgenden Beispiel daran, dass der run #2 Behälter beim Bestrahlen ganz oben aufgelegen ist und dadurch mit dem meisten Luftsauerstoff in Berührung gekommen ist. Dadurch sind in diesem Zellkulturbehälter wesentlich mehr Aldehyde und Ketone entstanden als bei dem run #1 Behälter, der während des Bestrahlunsgsprozesses weiter unten angeordnet war, und deshalb mit weniger Luftsauerstoff in Kontakt trat. Dieses Phänomen, das bei der Herstellung von Zellkulturbehältern häufig auftritt, konnte bisher weder erklärt noch beseitigt werden.

## Patentansprüche

1. Verfahren zur Herstellung eines mindestens teilweise aus Polymermaterial gefertigten Zellkulturbehälters für den Einmalgebrauch, wobei der Zellkulturbehälter eine äußere Schicht aus Polyethylen mit einer Dicke von 0,1 bis 0,5 mm sowie eine innere, mit der zu kultivierenden Zellkultur in Berührung kommende weitere Schicht aus Polyethylen oder EVA aufweist, die 0,1 bis 0,5 mm dick ist, wobei der Zellkulturbehälter zur Sterilisierung mit gamma-Strahlung in einer Bestrahlungsdosis von 25 bis 50 kGy behandelt wird, **dadurch gekennzeichnet, dass** der Zellkulturbehälter vor der Behandlung mit gamma-Strahlung mit Inertgas behandelt wird, wodurch die Menge an anwesendem Sauerstoff so gewählt wird, dass bei der Behandlung des Zellkulturbehälters mit gamma-Strahlung Aldehyde und Ketone in einer Konzentration von 0,01 bis 3 ppm entstehen, und wobei die Behandlung mit gamma-Strahlung unter Anwesenheit von 0,05 bis 15% Sauerstoff erfolgt.

2. Verfahren nach Anspruch 1, **dadurch gekennzeichnet, dass** der Zellkulturbehälter vor der Behandlung mit gamma-Strahlung mit Inertgas gefüllt und/oder in einen verschließbaren, mit Inertgas gefüllten Behälter eingebracht wird.

3. Zellkulturbehälter, herstellbar nach Anspruch 1 oder 2.

4. Zellkulturbehälter nach Anspruch 3, **dadurch gekennzeichnet, dass** er Aldehyde und/oder Ketone in einer Menge von 0,01 bis zu 3 ppm, insbesondere von 0,1 bis 1 ppm enthält.

5. Zellkulturbehälter nach Anspruch 3 oder 4, **dadurch gekennzeichnet, dass** mindestens in der innersten Lage UV-Stabilisatoren und/oder Anti-rads enthalten sind.

## Claims

1. A method for producing a single-use cell culture container, which is at least partially made of polymer material, wherein the cell culture container has an outer layer of polyethylene with a thickness of 0.1 to 0.5 mm as well as a further inner layer of polyethylene or EVA, which is contacted by the cell culture to be cultivated and has a thickness of 0.1 to 0.5 mm, wherein the cell culture container is treated with gamma radiation for sterilization at irradiation doses of 25 to 50 kGy, **characterised in that** the cell culture container is treated with inert gas before the treatment with gamma radiation, whereby the amount of oxygen present is selected such that during the treatment of the cell culture container with gamma radiation aldehydes and ketones are generated in a concentration of 0.01 to 3 ppm, and wherein the treatment with gamma radiation is carried out in the presence of 0.05 to 15% oxygen.

2. The method according to claim 1, **characterized in that** the cell culture container is filled with inert gas before gamma radiation treatment and/or introduced in a lockable container filled with inert gas before gamma radiation treatment.

3. A cell culture container producible according to claim 1 or 2.

4. The cell culture container according to claim 3, **characterized in that** it contains aldehydes and/or ketones in an amount of 0.01 up to 3 ppm, in particular 0.1 to 1 ppm.

5. The cell culture container according to claim 3 or 4, **characterized in that** UV stabilizers and/or anti-rads are contained at least in the innermost layer.

## Revendications

1. Procédé pour la préparation d'un récipient de culture cellulaire fabriqué au moins en partie avec un matériau polymère, le récipient de culture cellulaire présentant une couche externe en polyéthylène présentant une épaisseur de 0,1 à 0,5 mm, ainsi qu'une autre couche interne en polyéthylène ou en EVA (éthylène-acétate de vinyle) qui entre en contact avec la culture cellulaire devant être cultivée et présente une épaisseur de 0,1 à 0,5 mm, le récipient de culture cellulaire étant traité pour la stérilisation par un rayonnement gamma en une dose d'irradiation de 25 à 50 kGy, **caractérisé en ce que** le récipient de culture cellulaire est traité par un gaz inerte avant le traitement par un rayonnement gamma, moyennant quoi la quantité d'oxygène présent est choisie de sorte que lors du traitement du récipient de culture cellulaire avec un rayonnement gamma, des aldéhydes et des cétones se forment en une concentration de 0,01 à 3 ppm, et le traitement avec un rayonnement gamma étant effectué en présence de 0,05 à 15% d'oxygène.

2. Procédé selon la revendication 1, **caractérisé en ce que** le récipient de culture cellulaire est rempli avec un gaz inerte avant le traitement avec un rayonnement gamma et/ou est déposé dans un récipient refermable rempli de gaz inerte.

3. Récipient de culture cellulaire pouvant être préparé selon la revendication 1 ou 2.

4. Récipient de culture cellulaire selon la revendication 3, **caractérisé en ce qu'**il contient des aldéhydes et/ou des cétones en une quantité de 0,01 jusqu'à 3 ppm, en particulier de 0,1 jusqu'à 1 ppm.

5. Récipient de culture cellulaire selon la revendication 3 ou 4, **caractérisé en ce qu'**au moins la couche la plus à l'intérieur contient des stabilisateurs UV et/ou des agents anti-radiation.
